# EUROPEAN PATENT SPECIFICATION

(11) **EP 2 718 393 B1**
(45) Date of publication and mention of the grant of the patent: **05.08.2015**
(21) Application number: 12795095.4
(22) Date of filing: 25.10.2012
(51) Int. Cl.: C09K 9/02, C07D 491/107, C07D 209/12

(54) **NEW PHOTOCHROMIC COMPOUNDS, METHOD OF PRODUCTION THEREOF AND INTERMEDIATE COMPOUNDS**
NEUE PHOTOCHROME VERBINDUNGEN, VERFAHREN ZU IHRER HERSTELLUNG UND ZWISCHENPRODUKTE DAVON
NOUVEAUX COMPOSÉS PHOTOCHROMES, LEUR PROCÉDÉ DE PRODUCTION ET COMPOSÉS INTERMÉDIAIRES

(30) Priority: 30.08.2012 LT 2012082
(43) Date of publication of application: 16.04.2014
(73) Proprietor: Kaunas University of Technology, 44249 Kaunas (LT)
(72) Inventor: SACKUS, Algirdas, 44326 Kaunas (LT); MARTYNAITIS, Vytas, 51390 Kaunas (LT); VENGRIS, Mikas, 10212 Vilnius (LT); REDECKAS, Kipras, 05208 Vilnius (LT); DAGILIENE, Migle, LT-53268 Garliava, Kauno raj. (LT)
(74) Representative: Gerasimovic, Liudmila
(86) International application number: PCT/LT2012/000006
(87) International publication number: WO 2014/035224

(56) References cited:
- LUCAS HAUSER ET AL: "Reversible photochromism of polynorbornenes bearing spiropyran side groups", MONATSHEFTE FÜR CHEMIE - CHEMICAL MONTHLY ; AN INTERNATIONAL JOURNAL OF CHEMISTRY, SPRINGER-VERLAG, AU, vol. 143, no. 11, 29 August 2012 (2012-08-29), pages 1551-1558, XP035124154, ISSN: 1434-4475, DOI: 10.1007/S00706-012-0827-0
- KEUM S R ET AL: "Deuterated dicondensed indolinobenzospiropyran formed from the reaction of Fischer base-d2 and salicylaldehyde: Mechanism involving a carbinol intermediate", DYES AND PIGMENTS, ELSEVIER APPLIED SCIENCE PUBLISHERS. BARKING, GB, vol. 80, no. 1, 1 January 2009 (2009-01-01), pages 26-29, XP025479401, ISSN: 0143-7208, DOI: 10.1016/J.DYEPIG.2008.04.004 [retrieved on 2008-04-30]
- JENNIFER M. GALVIN ET AL: "Preparation and characterization of mixed thin films containing spiropyrans and long chain alkyl silanes: towards a command surface for liquid crystal realignment", SUPRAMOLECULAR SCIENCE, vol. 5, no. 1-2, 1 May 1998 (1998-05-01), pages 89-100, XP055067157, ISSN: 0968-5677, DOI: 10.1016/S0968-5677(97)00067-9

## Description

### Technical Field

The present invention is concerned with new spiro-condensed pyran derivatives, which possess enhanced photochromic properties and can be used as molecular photoswitches.

The invention also describes method of production of said new photochromic compounds and intermediates for their preparation as well.

### Background Art

Photochromism of indolino spiropyrans is well known [V.I. Minkin, Photoswitchable molecular systems based on spiropyrans and spirooxazines, 37-80 pp. In Molecular switches / edited by B. L. Feringa and W. R. Browne. Weinheim, Wiley-VCH, 2011, Vol. 1-2; Spiropyrans. R. C. Bertelson, 11-84 pp. In Organic Photochromic and Thermochromic Compounds, Vol. 1-2, Eds. J. C. Crano, R. J. Guglielmetti, Plenum Press. New York and London, 1999, Vol. 1-2.]

Photochromic compounds - indolino spiropyrans are also described in patents, for example, EP0411884A1, GB1270928A, JP2006249622A, US5155230, US5241075 etc. The coloured form of known spiropyrans appears in two stages process. The first stage, concerned with C-O bond breaking under the action of UV radiation, occurs in picoseconds; and the second stage, concerned with double bond cis-trans isomerization, appears in microseconds.

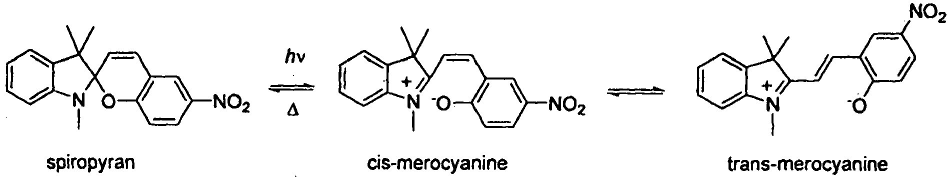

The thermal transformation of coloured merocyanine to spiropyran is retarded by the stage of trans-cis reisomerization, when compound returns to its initial state. For example, merocyanines return to spiropyran form during minutes, rate constant being ∼25×10⁻⁴ s⁻¹ in acetonitrile at 25 °C temperature. The generation of coloured form of indolino spiropyrans is fast enough, but due to thermo- and photodestruction, their operation possibilities are lower, besides the indolino spiropyrans are sensitive to oxygen and this restricts their application. [V. Matatesta, Photodegradation of Organic Photochromes In Organic Photochromic and Thermochromic Compounds. Vol.2 Eds. J. C. Crano and R. J. Guglielmetti. Kluwer Academic/Plenum Publishers, New York, 1991, 473 pp.]. Moreover, merocyanine form of spiropyrans easily hydrolyses in aqueos media. [Stafforst, T., and Hilvert, D. Kinetic characterization of spiropyrans in aqueous media. Chem. Commun. 2009, 287-288].

Similar compounds are disclosed in **D1 to D3.**

Remark: Compound 3 in D1 is wrongly drawn, as is obvious from reference 39 in D1, which is cited as D3 in this specification. The correct structure is shown by compound 11 c in D3.
**D1**
   LUCAS HAUSER ET AL: "Reversible photochromism of polynorbornenes bearing spiropyran side groups", MONATSHEFTE FÜR CHEMIE - CHEMICAL MONTHLY ; AN INTERNATIONAL JOURNAL OF CHEMISTRY, SPRINGER-VERLAG, AU, vol. 143, no. 11, 29 August 2012 (2012-08-29), pages 1551-1558, ISSN: 1434-4475, DOI: 10.1007/S00706-012-0827-0
**D2**
   KEUM S R ET AL: "Deuterated dicondensed indolinobenzospiropyran formed from the reaction of Fischer base-d2 and salicylaldehyde: Mechanism involving a carbinol intermediate", DYES AND PIGMENTS, ELSEVIER APPLIED SCIENCE PUBLISHERS. BARKING, GB, vol. 80, no. 1, 1 January 2009 (2009-01-01), pages 26-29, XP025479401, ISSN: 0143-7208, DOI: 1b.1016/J.DYEPIG.2008.04.004
**D3**
   JENNIFER M. GALVIN ET AL: "Preparation and characterization of mixed thin films containing spiropyrans and long chain alkyl silanes: towards a command surface for liquid crystal realignment", SUPRAMOLECULAR SCIENCE, vol. 5, no. 1-2, 1 May 1998 (1998-05-01), pages 89-100, DOI: 10.1016/S0968-5677(97)00067-9

At present there is a need in new materials that could respond to photostimulus and return to the initial state considerably faster, at the same time being stable and nonsensitive to environmental oxygen.

### Disclosure of the Invention

The aim of present invention is synthesis of new photochromic compounds, that act by opening and closing of 3,4-dihydro[2*H*]pyran ring, which is part of 1',3,3',4-tetrahydrospiro[chromen-2,2'-indole], which compounds are showing considerably higher rate of opening/closing cycle comparing with known spiropyrans.

To achieve this goal of the invention compounds of general structure (I) are proposed 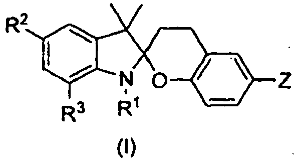 wherein
R¹ is C₁-C₃ alkyl, ω-functionalized alkyl, allyl or benzyl;
R² is hydrogen, methyl, F, Cl, Br, phenyl, 2-phenylethen-1-yl, phenylethinyl;
R³ is hydrogen or methyl;
Z is nitro group.

More specifically, the present invention covers compounds of the general formula (I), wherein R¹ is methyl, ethyl, allyl or benzyl; R² is hydrogen, methyl or bromine; R³ is hydrogen or methyl, Z is nitro group.

Preferred examples of new compounds are:
1',3',3'-trimethyl-6-nitro-1',3,3',4-tetrahydrospiro[chromen-2,2'-indole] **(4a),**
1',3',3',5'-tetramethyl-6-nitro-1',3,3',4-tetrahydrospiro[chromen-2,2'-indole] **(4b),**
5'-bromo-1',3',3'-trimethyl-6-nitro-1',3,3',4-tetrahydrospiro[chromen-2,2'-indole]**(4c),**
1',3',3',7'-tetramethyl-6-nitro-1',3,3',4-tetrahydrospiro[chromen-2,2'-indole] **(4d),**
1'-ethyl-3',3'-dimethyl-6-nitro-1',3,3',4-tetrahydrospiro[chromen-2,2'-indole] **(4e),**
1'-allyl-3',3'-dimethyl-6-nitro-1',3,3',4-tetrahydrospiro[chromen-2,2'-indole] **(4f),**
1'-benzyl-3',3'-dimethyl-6-nitro-1',3,3',4-tetrahydrospiro[chromen-2,2'-indole] **(4g).**

Compounds of the invention are exhibiting photochromic properties. They can be used as molecular photoswitches.

Another object of the invention are compounds of general structure (III), 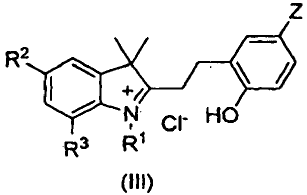 wherein R¹ is methyl or ethyl; R² is hydrogen, methyl or bromine; R³ is hydrogen or methyl, and Z is nitro group, which compounds are intermediates for obtaining photochromic compounds of present invention.

Yet another object of present invention is a method of production of the new compounds of formula (I), 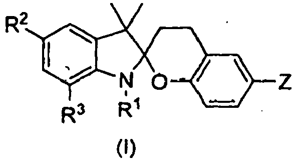 wherein R¹ is methyl, ethyl, allyl or benzyl; R² is hydrogen, methyl or bromine; R³ is hydrogen or methyl, and Z is nitro group, said method comprising alkylating of 1-substituted-3,3-dimethyl-2-methylenindole derivatives with 2-chloromethyl-4-nitrophenol to obtain corresponding 3*H*-indolium salts, which further are treated under basic conditions.

This method is specified in comprising the following steps:
a) alkalizing a solution of corresponding 3*H*-indolium salt to obtain enamine of general formula (II) 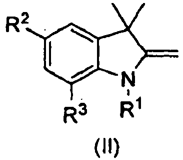
b) treating the obtained enamine of formula (II) with 2-chloromethyl-4-nitrophenol to obtain a chloride of general formula (III) 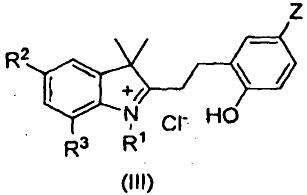
c) purifying the chloride of formula (III), obtained in step ii) by recrystallization or chromatografically, following by optional dissolution and precipitation under neutral or slightly basic conditions to obtain the target compounds of formula (I);
d) if desired, recrystallizing the target compounds of formula (I) from acetonitrile.

To obtain compounds of formula (I), wherein R¹ is methyl, ethyl or benzyl; R² is hydrogen, methyl or bromine; R³ is hydrogen or methyl, and Z is nitro group, precipitation in step iii) is carried out by adding dropwise an aqueous solution of base, for example, ammonia; or, to obtain compounds of formula (I) wherein R¹ is allyl, R²-R⁴ is hydrogen and Z is nitro group, no base (ammonia) is added in step iii)

The method is also specific in that: in the i) step as the suitable 3*H*-indolium salt is used, for example, iodide or perchlorate; sodium carbonate is used for alkalization; and the reaction mixture in step ii) is stirred for up to 3 hours at 20°C-80°C temperature and then optionally is kept at 5°C ± 1°C temperature for at least 12 hours.

### Detailed Description of the Invention

New class of photochromic compounds is developed, comprising derivatives of 1',3,3',4-tetrahydrospiro[chromen-2,2'-indoles] of general formula (I).

Substituted derivatives of 3,4-dihydro-2*H*-pyran of present invention were found to have a general structure **A,** covering two heterocyclic indole and 3,4-dihydro[2*H*]chromen systems, connected through a spiro atom and positioned perpendicularly to each other.

The structure with assymetric carbon atom in compounds of general formula (I), even having similar substituents as in spiropyran B, is different in that there is no attached C=C double bond as in structure B. Instead, there is saturated C-C bond in structure A.

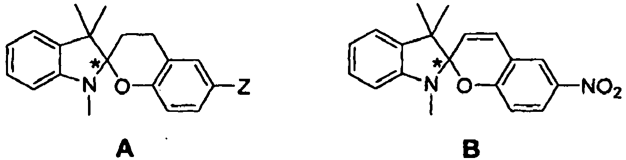

The main specific feature of invented structure was found to be that under the action of ultraviolet radiation and breaking of C-O bond in 3,4-dihydro[2*H*]pyran ring of A structure, no cis/trans and trans/cis isomerization, comparing with spiropyran B, occurs. As a result slow (of microseconds order) trans/cis isomerization in the second stage of fotochromic cycle, typical for B structure, is avoided in the compounds of structure A.

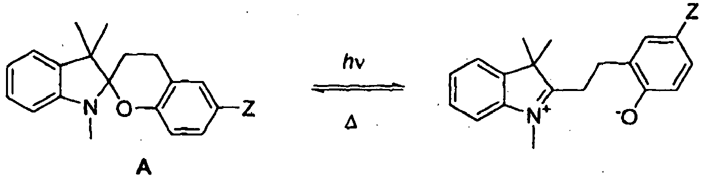

It was found by the inventors, that when solutions of compounds described in this patent were irradiated with monochromatic ultraviolet radiation [for example, Nd:YAG laser (EKSPLA NL30); third harmonic; vawelength - 355 nm; pulse duration - 5 ns; pulse energy - 3 mJ], heterolytic cleavage of bond between chiral center and oxygen atom in the molecule of 1',3,3',4-tetrahydrospiro[chromen-2,2'-indole] occurs and compound having open chain structure, possesing 3*H*-indolium cation moiety and 4-nitrophenolate anion moiety interconnected through 2 carbon atoms chain appears. After switching irradiation off the coloured form of compounds thermally returns to the initial colourless state, after connection of oxygen atom from phenolate anion to α-carbon atom of indole ring system. Nevertheless known derivatives of indolino spiropyrans and compounds according to present invention - 1',3,3',4-tetrahydrospiro[chromen-2,2'-indoles] seem to have some structural similarity, however the rate of cycle opening/closing was found higher in the case of 1',3,3',4-tetrahydrospiro[chromen-2,2'-indoles]. Besides the latter compounds are able to work in usual environment without oxidative destruction. Compounds of this invention show high photostability.

Compounds described in this patent application were synthesized by β-C alkylating of 1,3,3-trimethyl-2-methylen-2,3-dihydro-1*H*-indole and its derivatives with 2-chloromethyl-4-nitrophenol and subsequent spirocyclization of corresponding salts into 1',3,3',4-tetrahydrospiro[chromen-2,2'-indole] derivatives.

Said 1',3,3',4-tetrahydrospiro[chromen-2,2'-indole] derivatives were synthesized according to scheme 1 below:

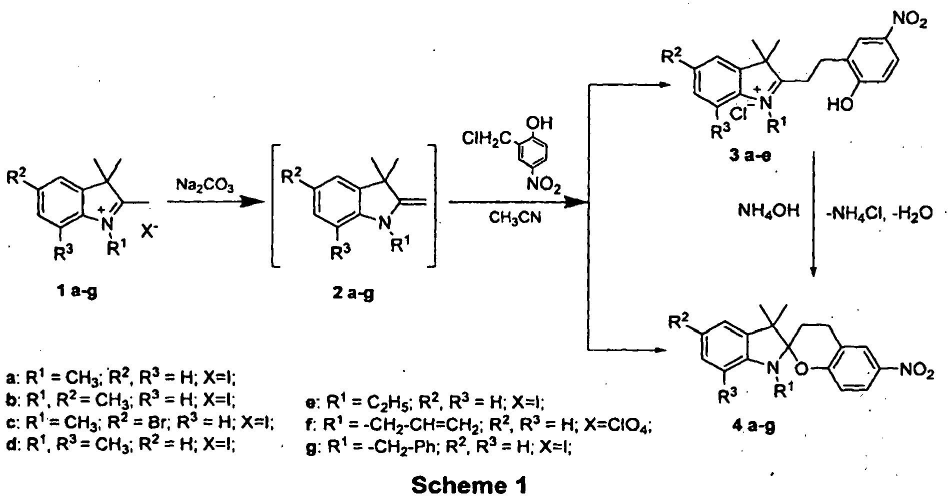

### Modes for Carrying out the Invention

Information on examples of real embodiments is provided below, describing the modes of preparation compounds of present invention **(4a-g)** and properties thereof. This information is provided for the illustrative purpose and is not limiting the scope of present invention.

### Example 1

### 1',3',3'-Trimethyl-6-nitro-1',3,3',4-tetrahydrospiro[chromen-2,2'-indole] (4a)

To a stirred aqueous solution (15 ml) of 3*H*-indolium salt **1a** (5 mmol) sodium carbonate (1.06 g, 10 mmol) was added. The solution became turbid and the reaction mixture was extracted with diethyl ether (3 × 20 ml). Combined organic layer was dried over unhydrous sodium sulfate and solvent was evaporated under reduced pressure, yielding the corresponding enamine. The obtained enamine **2a** was dissolved in acetonitrile (3 ml), 2-chloromethyl-4-nitrophenol (938 mg, 5 mmol) was added to the solution and the reaction mixture was stirred for 3 h at 20 °C temperature and then held at 5 °C temperature for 12 h. The formed crystals were filtered yielding corresponding [2-(2-hydroxy-5-nitrophenyl)ethyl-1]-3*H*-indolium chloride **3a.** Obtained chloride **3a** (intermediate compound) was recrystallized from ethanol.

*[2-(2-Hydroxy-5-nitrophenyl)ethyl-1]-1,3,3-trimethyl-3H-indolium chloride **3a.*** Yield 39%, white crystals, m.p. 208-211 °C. ¹H NMR (300 MHz, DMSO-*D*₆): δ 1.61 (s, 6H, 2×3'-CH₃), 2.99-3.05 (m, 2H, CH₂), 3.33-3.41 (m, 2H, CH₂), 4.13 (s, 3H, N-CH₃), 7.26 (d, J = 9.0 Hz, 1H, 3-H-Ph), 7.63-7.66 (m, 2H, Ar-H), 7.83-7.86 (m, 1H, Ar-H), 7.95-7.98 (m, 1H, Ar-H), 8.09 (dd, J = 9.0 Hz, J = 2.8 Hz, 1 H, 4-H-Ph), 8.35 (d, *J* = 2.8 Hz, 1H, 6-H-Ph), 11.98 (s, 1H, OH). ¹³C NMR (75 MHz, DMSO): δ 21.2 (2×CH₃), 26.2, 26.4, 35, 54.4, 115.3, 115.4, 123.2, 124.7, 126.4, 126.6, 128.8, 129.6, 139.3, 141.7, 142.1, 162.2, 195.6. IR (KBr, cm⁻¹): v _{O-H} = 3416; v _{C-H arom} = 3041; v _{C-H aliph} = 2976; v _{NO2 asim} = 1517; v _{NO2 sim} = 1347. MS m/z (%): 325 (M-Cl⁻, 100). Anal. Calcd. For C₁₉H₂₁ClN₂O₃, %: C 63.24; H 5.87; N 7.76. Found: C 62.84; H 5.99; N 7.75.

Intermediate compound **3a** (3 mmol) was dissolved in diluted ethanol (ethanol/water 1:2 v/v) and aqueous solution of ammonia (10%) was added dropwise to the solution till solution became turbid. The mixture was extracted with ether (3 × 20 ml), combined extract was dried over unhydrous sodium sulfate and solvent was evaporated under reduced pressure. Obtained compound **4a** was recrystallized from acetonitrile.

*1'*,*3'*,*3*'-*Trimethyl*-*6*-*nitro*-*1'3*,*3*',*4*-*tetrahydrospiro[chromen-2,2'-indole] **4a.*** Yield 74%, yellow crystals, m.p. 187-189 °C. ¹H NMR (300 MHz, CDCl₃): δ 1.26 (s, 6H, 2×3'-CH₃), 2.32-2.41 (m, 2H, CH₂), 2.86 (s, 3H, N-CH₃), 3.00-3.16 (m, 2H, CH₂), 6.60 (d, *J* = 7.5 Hz, 1H, 7'H), 6.78 (d, *J* = 9.0 Hz, 1H, 8H), 6.85 (dt, *J* = 7.5 Hz, *J* = 0.9 Hz, 1H, 5'H), 7.06 (dd, *J* = 7.5 Hz, *J* = 0.9 Hz, 1H, 4'H), 7.20 (dt, *J* = 7.5 Hz, *J =* 0.9 Hz, 1H, 6'H), 7.98 (dd, *J =* 9.0 Hz, *J =* 2.7 Hz, 1H, 7H), 8.04 (d, *J* = 2.7 Hz, 1H, 5H). ¹³C NMR (75 MHz, CDCl₃): δ 21.9, 23.3, 24, 25.6, 28.3, 49.4, 104.3, 107.2, 116.7, 119.2, 121.2, 121.4, 124.1, 125.1, 127.8, 136.8, 140.4, 148.4, 161.8. IR (KBr, cm⁻¹): v _{C-H} arom = 3068; v _{C-H aliph} = 2965; v _{NO2 asim} = 1509; v _{NO2 sim} = 1329. MS m/z (%): 325 (M + H⁺, 30). Anal. Calcd. for C₁₉H₂₀N₂O₃, %: C 70.35; H 6.21; N 8.64. Found: C 70.66; H 6.33; N 8.66.

### Example 2

### 1',3',3',5'-Tetramethyl-6-nitro-1',3,3',4-tetrahydrospiro[chromen-2,2'-indole](4b)

Synthesis was carried out according to scheme 1 and procedure described in Example 1 was used for preparation of target compound via an intermediate compound **3b.**

*[2-(2-Hydroxy-5-nitrophenyl)ethyl-1]-1,3,3,5-tetramethyl-3H-indolium chloride **3b.*** Yield 45%, white crystals, m. p. 190-194 °C. ¹H NMR (300 MHz, DMSO-*D*₆): δ 1.58 (s, 6H, 2×3'-CH₃), 2.45 (s, 3H, 5-CH₃), 2.99-3.05 (m, 2H, CH₂), 3.32-3.38 (m, 2H, CH₂), 4.1 (s, 3H, N-CH₃), 7.27 (d, J = 9 Hz, 1H, 3-H-Ph), 7.45 (d, J = 8.25 Hz, 1H, 6-H-Ar), 7.66 (s, 1H, 4-H-Ar), 7.83 (d, *J* = 8.25 Hz, 1H, 7-H-Ar), 8.07 (dd, J = 9 Hz, *J* = 3 Hz, 1H, 4-H-Ph), 8.34 (d, *J =* 3 Hz, 1 H, 6-H-Ph), 11,98 (s, 1H, OH). ¹³C NMR (75 MHz, DMSO): δ 21, 21.3 (2×CH₃), 26.2, 26.3, 35, 54.1, 115, 115.3, 123.6, 124.6, 126.4, 126.5, 129.2, 139.3, 139.8, 140, 141.8, 162.2, 194.4. **IR** (KBr, cm⁻¹): v _{O-H} = 3394; v _{C-H} arom = 3029; v _{C-H aliph} = 2975; v _{NO2 asim} = 1523; v _{NO2 sim} = 1337. MS m/z (%): 339 (M-Cl⁻, 90). Anal. Calcd. for C₂₀H₂₃ClN₂O₃, %: C 64.08; H 6.18; N 7.47. Found: C 63.51; H 6.33; N 7.39.

*1',3',3',5'-Tetramethyl-6-nitro-1',3,3',4-tetrahydrospiro[chromen-2,2'-indole] **4b.*** Yield 80%, yellow crystals, m. p. 178-179 °C. ¹H NMR (300 MHz, CDCl₃): δ 1.25 (s, 6H, 2×3'-CH₃), 2.32 (s, 3H, 5'-CH₃), 2.36 (br.s, 2H, CH₂), 2.83 (s, 3H, N-CH₃), 3.05 (br.s, 2H, CH₂), 6.5 (d, *J* = 8.1 Hz, 1H, 7'H); 6.78 (d, *J =* 9 Hz, 1H, 8H), 6.86-6.88 (m, 1H, 4'H), 6.98-7.02 (m, 1H, 6'H), 7.97 (dd, J = 9 Hz, *J* = 2.7 Hz, 1H, 7H), 8.03 (d, *J* = 2.7 Hz, 1H, 5H). ¹³C NMR (75 MHz, CDCl₃): δ 20.9, 21.9, 23.4, 24.1, 25.6, 28.5, 49.5, 104.7, 107.1, 116.7, 121.4, 122.1, 124.1, 125, 128.1, 128.6, 137, 140.5, 146.3, 161.9. IR (KBr, cm⁻¹): v _{C-H arom} = 3065; v _{C-H aliph} = 2940; v _{NO2 asim} = 1499; v _{NO2 sim} = 1332. MS m/z (%): 339 (M + H⁺, 100). Anal. Calcd. for C₂₀H₂₂N₂O₃, %: C 70.99; H 6.55; N 8.28. Found: C 70.5; H 6.6; N 8.45.

### Example 3

### 5'-Bromo-1',3',3'-trimethyl-6-nitro-1',3,3',4-tetrahydrospiro[chromen-2,2'-indole] (4c).

Synthesis was carried out according to procedure described in the example 1 through intermediate compound **3c.**

*5-Bromo-[2-(2-hydroxy-5-nitrophenyl)ehyl-1]-1,3,3-trimethyl-3H-indolium chloride* 3c. Yield 62%, white crystals, m. p. 210-215 °C. ¹NMR (300 MHz, DMSO-D₆): δ 1.62 (s, 6H, 2×3'-CH₃), 2.97-3.08 (m, 2H, CH₂), 3.33-3.42 (m, 2H, CH₂), 4.11 (s, 3H, N-CH₃), 7.24 (d, J = 8.9 Hz, 1H, 3-H-Ph), 7.85-7.94 (m, 2H, 6-H-Ar, 4-H-Ar), 8.07 (dd, *J* = 8.9 Hz, J = 2.1 Hz, 4-H-Ph), 8.18 (s, 1H, 7=H-Ar), 8.34 (d, J = 2.1 Hz, 1H, 6-H-Ph), 11,98 (s, 1H, OH). ¹³C NMR (75 MHz, DMSO): δ 21 (2×CH₃), 26.1, 26.6, 35.2, 54.7, 115.3, 117.4, 123, 124.7, 126.4, 126.5, 126.6, 131.8, 139.3, 141.5, 144, 162.2, 196. IR (KBr, cm⁻¹): v _{O-H} = 3382; v _{C-H arom} =3032; v _{C-H aliph} = 2931; v _{NO2 asim} = 1520; v _{NO2 sim} = 1336. MS m/z (%): 403, 405 (M Cl⁻, 100). Anal. Calcd. for C₁₉H₂₀BrClN₂O₃, %: C 51.90; H 4.58; N 6.37. Found: C 50.88; H 4.5; N 6.31.

*5'-Bromo-1',3',3'-trimethyl-6-nitro-1',3,3',4-tetrahydrospiro[chromen-2,2'-indole]* **4c.** Yield 87%, yellow crystals, m. p. 133-136 °C. ¹H NMR (300 MHz, CDCl₃): δ 1.21 (s, 6H, 2×3'-CH₃), 2.24-2.42 (m, 2H, 3-CH₂), 2.82 (s, 3H, N-CH₃), 2.96-3.17 (m, 2H, 4-CH₂), 6.45 (d, *J* = 8.3 Hz, 1H, 7'H); 6.77 (d, *J* = 9 Hz, 1H, 8H), 7.12 (d, *J* = 2.1 Hz, 1H, 4'H), 7.27 (dd, *J* = 8.3 Hz, J = 2.1 Hz, 1H, 6'H), 7.98 (dd, *J* = 9.0 Hz, *J* = 2.7 Hz, 1H, 7H), 8.03 (d, J = 2.7 Hz, 1H, 5H). ¹³C NMR (75 MHz, CDCl₃): δ 21.7, 23.2, 23.9, 25.5, 28.4, 46.6, 104.1, 108.6, 111, 116.7, 121.2, 124.2, 124.5, 125.1, 130.5, 139.2, 140.6, 147.5, 161.5. IR (KBr, cm⁻¹): v _{C-H arom} = 3059; V _{C-H aliph} = 2977; v _{NO2 asim} = 1512; v _{NO2 sim} = 1329. MS m/z (%) 403, 405 (M + H⁺, 100). Anal. Calcd. for C₁₉H₁₉BrN₂O₃, %: C 56.59; H 4.75; N 6.95. Found: C 57.08; H 4.78; N 6.95.

### Example 4

### 1',3',3',7'-Tetramethyl-6-nitro-1',3,3',4-tetrahydrospiro[chromen-2,2'-indole] (4d).

Synthesis was carried out according to procedure described in the example 1, through intermediate compound **3d.**

*[2-(2-Hydroxy-5-nitrophenyl)ethyl-1]-1,3,3,7-tetramethyl-3H-indolium chloride **3d.*** Yield 48%, white crystals, m. p. 200-203 °C. ¹H NMR (300 MHz, DMSO-*D*₆): δ 1.58 (s, 6H, 2×3'-CH₃), 2.78 (s, 3H, 7-CH₃), 2.95-3.01 (m, 2H, CH₂), 3.32-3.38 (m, 2H, CH₂), 4.31 (s, 3H, N-CH₃), 7.31 (d, *J* = 8.9 Hz, 1H, 3-H-Ph), 7.39 (d, *J* = 7.5 Hz, 1H, 4-H-Ar), 7.5 (t, *J* = 7.5 Hz, 1H, 5-H-Ar), 7.67 (d, *J* = 7.5 Hz, 1H, 6-H-Ar), 8.07 (dd, *J* = 8.9 Hz, *J* = 2.5 Hz, 1 H, 4-H-Ph), 8.35 (d, *J* = 2.5 Hz, 1H, 6-H-Ph), 12.1 (s, 1H, OH). ¹³C NMR (75 MHz, DMSO): δ 19.1, 21.5 (2×CH₃), 26.2, 38.8, 39, 53.5, 115.3, 121.1, 124.6, 126.3, 126.6, 127.2, 129.3, 132.5, 139.2, 140.3, 142.8, 162.3, 195.3. IR (KBr, cm⁻¹): v _{O-H} = 3624; v _{C-H arom} = 3050; v _{C-H aliph} = 2923; v _{NO2 asim} = 1520; v _{NO2 sim} = 1345. MS m/z (%): 339 (M-Cl⁻, 100). Anal. Calcd. for C₂₀H₂₃ClN₂O₃, %: C 64.08; H 6.18; N 7.47. Found: C 64.45; H 6.25; N 8.1.

*1',3',3',7'-Tetramethyl-6-nitro-1',3,3',4-tetrahydrospiro[chromen-2,2'-indole]* **4d*.*** Yield 51%, yellow crystals, m. p. 206-209 °C. ¹H NMR (300 MHz, CDCl₃): δ 1.24 (s, 6H, 2×3'-CH₃), 2.31-2.36 (m, 2H, 3-CH₂), 2.5 (s, 3H, 7'-CH₃), 3.05-3.1 (m, 5H, 4-CH₂, N-CH₃), 6.76-6.82 (m, 2H, 5'-H, 8-H); 6.91-6.96 (m, 2H, 4'-H, 6'-H), 7.99 (dd, *J* = 9 Hz, *J=* 2.7 Hz, 1H, 7-H), 8.04 (d, *J* = 2.7 Hz, 1H, 5H). ¹³C NMR (75 MHz, CDCl₃): δ 20.1, 23.4, 24, 24.1, 25.6, 32, 48.8, 104.7, 116.7, 119.1, 119.2, 119.8, 121.5, 124.1, 126.1, 131.7, 137.6, 140.5, 146.5, 161.8. IR(KBr, cm⁻¹): v _{C-H arom} = 3084; v _{C-H aliph} = 2975; v _{NO2 asim} = 1513; v _{NO2 sim} = 1330. MS m/z (%): 339 (M + H⁺, 100). Anal. Calcd. for C₂₀H₂₂N₂O₃, %: C 70.99; H 6.55; N 8.28. Found: C 70.42; H 6.67; N 8.64.

### Example 5

### 1'-Ethyl-3',3'-dimetil-6-nitro-1',3,3',4-tetrahydrospiro[chromen-2,2'-indole] (4e)

Synthesis was carried out according to procedure described in the example 1, through intermediate compound **3e.**

*1-Ethyl-[2-(2-hydroxy-5-nitrophenyl)ethyl-1]-3,3-dimethyl-3H-indolium chloride* 3e. Yield 37%, white crystals, m. p. 195-199°C. ¹H NMR (300 MHz,DMSO-*D*₆): 1.55 (t, *J=* 6.9 Hz, 3H, CH₂CH₃), 1.64 (s, 6H, 2×3'-CH₃), 3.00-3.07 (m, 2H, CH₂), 3.33-3.44 (m, 2H, CH₂), 4.63-4.73 (m, 2H, CH₂CH₃), 7.27 (d, *J* = 9 Hz, 1H, 3-H-Ph), 7.64-7.67 (m, 2H, Ar-H), 7.86-7.89 (m, 1H, Ar-H), 8.02-8.05 (m, 1H, Ar-H), 8.08 (dd, *J =* 9 Hz, *J =* 2.7 Hz, 1H, 4-H-Ph), 8.38 (d, *J* = 2.7 Hz, 1H, 6-H-Ph), 12.01 (s, 1H, OH). ¹³C NMR (75 MHz, DMSO-*D*₆): δ 21.5 (2×CH₃), 26.5, 26.9, 30.1, 43.5, 54.9, 115.4, 115.9, 123.5, 124.7, 126.4, 126.6, 129, 129.7, 139.3, 140.6, 142.6, 162.2, 195.8. IR (KBr, cm⁻¹): v_{O-H} = 3462; v _{C-H arom} = 3050; v _{C-H aliph} = 2971; v _{NO2 asim} = 1520; v _{NO2 sim} = 1338. MS m/z (%): 339 (M-Cl⁻, 100). Anal. Calcd. for C₂₀H₂₃ClN₂O₃, %: C 64.08; H 6.18; N 7.47. Found: C 63.98; H 6.25; N 7.31.

*1'-Ethyl-3',3'-dimethyl-6-nitro-1',3,3'*,*4-tetrahydrospiro[chromen-2,2'-indole]* **4e.** Yield 30%, yellow crystals, m. p. 154-156 °C. ¹H NMR (300 MHz, CDCl₃): δ 1.23 (t, *J* = 7.2 Hz, 3H, CH₂CH₃), 1.25 (s, 6H, 2×3'-CH₃), 2.21-2.48 (m, 2H, CH₂CH₃), 2.95-3.44 (m, 4H, 2×CH₂), 6.58-6.61 (m, 1H, 7'-H), 6.76 (d, *J* = 9.3 Hz, 1 H, 8-H), 6.82 (dt, *J* = 7.5 Hz, *J* = 0.9 Hz, 1H, 5'-H), 7.06 (dd, *J* = 7.5 Hz, *J* = 0.9 Hz, 1H, 4'-H), 7.19 (dt, *J* = 7.5 Hz, *J* = 0.9 Hz, 1H, 6'-H), 7.98 (dd, *J* = 9 Hz, *J* = 2.7 Hz, 1 H, 7-H), 8.04 (d, *J* = 2.7 Hz, 1H, 5-H). ¹³C NMR (75 MHz, CDCl₃): δ 14.9, 21.8, 23.5, 24.7, 25.7, 37.3, 49.6, 104.5, 106.3, 116.7, 118.6, 121.3 (2×C), 124.1, 125.1, 127.8, 136.6, 140.3, 147.4, 161.8. IR (KBr, cm⁻¹): v _{C-H arom} = 3068; v _{C-H aliph} = 2970; v _{NO2 asim} = 1510; v _{NO2 sim} = 1331. MS m/z (%): 339 (M + H⁺, 50). Anal. Calcd. for C₂₀H₂₂N₂O₃, %: C 70.99; H 6.55; N 8.28. Found: C 70.57; H 6.61; N 8.24.

### Example 6

### 1'-Allyl-3',3'-dimethyl-6-nitro-1',3,3',4-tetrahydrospiro[chromen-2,2'-indole] (4f).

Synthesis was carried out according to procedure described in the example 1, with the difference in that after stirring reaction mixture for 3 h at 20 °C temperature and storing for 12 h at 5 °C temperature; the corresponding target 1',3,3',4-tetrahydrospiro[chromen-2,2'-indole] 4f was obtained directly, and the formed crystals thereof were filtered.

*1'-Allyl-3',3'-dimethyl-6-nitro-1',3,3',4-tetrahydrospiro[chromen-2,2'-indole]* **4f**. Yield 28%, yellow crystals, m. p. 160-161 °C. ¹H NMR (300 MHz, CDCl₃): δ 1.29 (s, 6H, 2×3'-CH₃), 2.35 (br.s, 2H, 3-CH₂), 3.06 (br.s, 2H, N-CH₂), 3.88 (br.s, 2H, 4-CH₂), 5.14 (dq, *J* = 10.2 Hz, *J =* 1.8 Hz, 1H, =CH₂) 5.27(dq, *J* = 17.1 Hz, *J =* 1.8 Hz, 1H, =CH₂), 5.94 (ddt, *J* = 17.1 Hz, *J* = 10.2 Hz, *J* = 4.5 Hz, 1 H, -CH=), 6.56 (d, *J* = 7.5 Hz, 1H, 7'-H), 6.78 (d, *J =* 9 Hz, 1H, 8-H), 6.85 (t, *J* = 7.5 Hz, 1H, 5'-H), 7.07 (d, *J =* 7.5 Hz, 1H, 4'-H), 7.17 (dt, *J* = 7.5 Hz, *J* = 1.2 Hz, 1H, 6'-H), 7.98 (dd, *J* = 9 Hz, *J* = 3 Hz, 1H, 7-H), 8.04 (d, *J* = 3 Hz, 1H, 5-H). ¹³C NMR (75 MHz, CDCl₃): δ 21.8, 23.4, 24.7, 25.9, 45.5, 49.6, 104.4, 107.1, 115.1, 116.7, 119.1, 121.1, 121.2, 124.1, 125.1, 127.8, 135.0, 136.6, 140.5, 147.7, 161.7. IR (KBr, cm⁻¹): v _{C-H arom} = 3031; v _{C-H aliph} = 2935; v _{NO2 asim} = 1511; v _{NO2 sim} = 1339. Anal. Calcd. for C₂₁H₂₂N₂O₃, %: C 71.98; H 6.33; N 7.99. Found: C 71.97; H 6.25; N 8.12.

### Example 7

### 1'-Benzyl-3',3'-dimethyl-6-nitro-1',3,3',4-tetrahydrospiro[chromen-2,2'-indole] (4g).

Synthesis was out carried according to procedure described in the example 1, with the difference that after stirring the reaction mixture for 3 h at 80 °C temperature; the target compound **4g** was obtained directly. This compound was not possible to recrystallize and column chromatography on silica gel (60 Merck F254, eluent n-hexane/acetone 6/1) was used for purification.

*1'-Benzyl-3',3'-dimethyl-6-nitro-1',3,3',4-tetrahydrospiro[chromen-2,2'-indole]* **4g**. Yield 29%, yellow crystals, m. p. 62-64 °C. ¹H NMR (300 MHz, CDCl₃): δ 1.37 (s, 6H, 2×3'-CH₃), 2.35-2.42 (m, 2H, 3-CH₂), 3.02-3.06 (m, 2H, 4-CH₂), 4.55 (s, 2H, N-CH₂), 6.51-6.53 (m, 1H, 7'-H), 6.83-6.93 (m, 2H, 5'-H, 8-H), 7.28-7.32 (m, 2H, 6'-H, 4'-H), 7.34-7.39 (m, 5H, Ph-H), 7.98-8.02 (m, 2H, 7-H, 5-H). ¹³C NMR (75 MHz, CDCl₃): δ 23.6, 23.7, 24.5, 25.2, 47.2. 50.1, 107.9. 116.7, 118.0, 120.1, 121.4, 122.5, 124.2, 125.1, 126.1, 126.5, 127.1, 127.5, 128.0, 128.7, 137.0, 138.7, 140.5, 147.6, 161.7. IR (KBr, cm⁻¹): v _{C-H arom} = 3061; v _{C-H aliph} = 2966; v _{NO2 asim} = 1516; v _{NO2 sim} = 1337. MS m/z (%): 401 (M + H⁺, 100). Anal. Calcd. for C₂₅H₂₄N₂O₃, %: C 74.98; H 6.04; N 7.0. Found: C 74.5; H 6.14; N 6.65.

Photochromic properties of target compounds **4a-g** were determined from solutions in acetonitrile (10⁻⁵ M/I). Steady state UV-Vis absorption spectra were recorded on scanning spectrometer (Shimadzu UV-3101PC). Flash photolysis experiments were performed with Nd:YAG laser 3-rd harmonic radiation (EKSPLA NL30, pulse energy 3 mJ per pulse, λ = 355 nm, pulse duration 5 ns). A solution of benzopherione (0.5 OD at 355 nm) in MeCN, optically adjusted for wavelength of excitation, was used to measure quantum yields.

Quantum yield of photochromic transition to opened form was evaluated according to energetic characteristics (the growth of difference absorbance signal vs exciting pulse energy) and molar extinction coefficient.

Results of the measurements are provided in the Table 1.

**Table 1**

| Results of photochromic measurements | | | | |
|---|---|---|---|---|
| Compound | Structural formula | Absorption maximum in the visible spectrum of opened form, λₘₐₓ (nm) | Characteristic relaxation time, τ (ns) | Quantum yield, Φ (%) |
| **4a** | 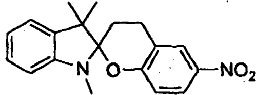 | 440 | 22 | 5,5 |
| **4b** | 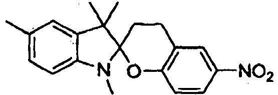 | 450 | 22 | 5,6 |
| **4c** | 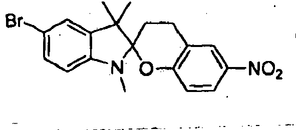 | 480 | 25 | 10,0 |
| **4d** | 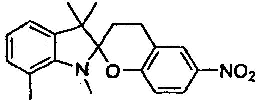 | 450 | 29 | 4,9 |
| **4e** | 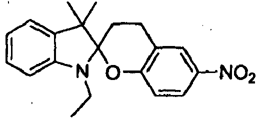 | 450 | 36 | 5,8 |
| **4f** | 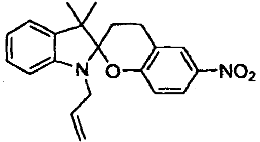 | 450 | 37 | 5,7 |
| **4g** | 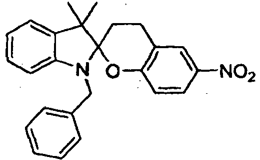 | 450 | 41 | 6,4 |

### Industrial Applicability

The data on photochromic properties presented in the Table 1 show that compounds of present invention can be used as molecular photoswitches, in which under UV radiation opening of 3,4-dihydro-2*H*-pyran ring occurs and coloured form of compound is generated, with subsequent return to initial non-coloured form. The total duration of formation of coloured form and returning back to non-coloured form is in the range of 22-41 ns. This process is repeatable many times; solutions of these compounds remain photostable and without noticable destruction while operating more than 2000 cycles in the air.

In general, the present invention has the following main advances comparing with the known state of the art:
1. The total duration of coloured form formation and returning back to the initial non-coloured state of 1',3,3',4-tetrahydrospiro[chromen-2,2'-indoles] of present invention is shorter than in well-studied indolino spiropyrans.
2. Compounds of the present invention possess higher stability to photodestruction and are able for switching for more than 2000 cycles.
3. These compounds are non-sensitive to the oxygen in the air and can be used in environment from which oxygen is not removed.

## Claims

1. Compounds of the general formula (I) 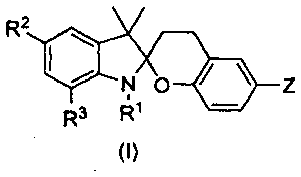 wherein
R¹ is C₁-C₃₋ alkyl, ω-functionalized alkyl, allyl or benzyl;
R² is hydrogen, methyl, F, Cl, Br, phenyl, 2-phenylethen-1-yl or phenylethinyl;
R³ is hydrogen or methyl;
Z is nitro group.

2. The compounds of the general formula (I) according to claim 1, wherein
R¹ is methyl, ethyl, allyl or benzyl; R² is hydrogen, methyl or Br; R³ is hydrogen or methyl, Z is nitro group.

3. The compound according to claim 1 or 2, which is 1',3',3'-trimethyl-6-nitro-1',3,3',4-tetrahydrospiro[chromen-2,2'-indole] **(4a).**

4. The compound according to claim 1 or 2, which is 1',3',3',5'-tetramethyl-6-nitro-1',3,3',4-tetrahydrospiro[chromen-2,2'-indole] **(4b).**

5. The compound according to claim 1 or 2, which is 5'-brom-1',3',3'-trimethyl-6-nitro-1',3,3',4-tetrahydrospiro[chromen-2,2'-indole] **(4c).**

6. The compound according to claim 1 or 2, which is 1',3',3',7'-tetramethyl-6-nitro-1',3,3',4-tetrahydrospiro[chromen-2,2'-indole] **(4d).**

7. The compound according to claim 1 or 2, which is 1'-ethyl-3',3'-dimethyl-6-nitro-1',3,3',4-tetrahydrospiro[chromen-2,2'-indole] **(4e).**

8. The compound according to claim 1 or 2, which is 1'-allyl-3',3'-dimethyl-6-nitro-1',3,3',4-tetrahydrospiro[chromen-2,2'-indole] **(4f).**

9. The compound according to claim 1 or 2, which is 1'-benzyl-3',3'-dimethyl-6-nitro-1',3,3',4-tetrahydrospiro[chromen-2,2'-indole] **(4g).**

10. The compound according to any one of preceeding claims, which is exhibiting photochromic properties.

11. Use of the compounds according to any of 1-9 claims as molecular photoswitches.

12. Compounds of the general formula (III), 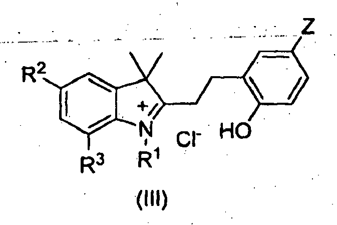 wherein R¹ is methyl or ethyl; R² is hydrogen, methyl or Br; R³ is hydrogen or methyl and Z is nitro group, as intermediate compounds for production of compounds defined in claims 3-7.

13. A method for production of compounds of formula (I) 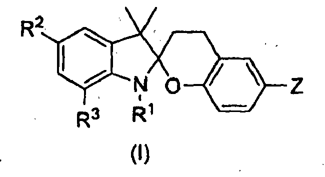 according to any of claims 1-9, wherein R¹ is methyl, ethyl, allyl or benzyl; R² is hydrogen, methyl or Br; R³ is hydrogen or methyl, and Z is nitro group, which method comprises alkylating of 1-substituted-3,3-dimethyl-2-methylenindole derivatives with 2-chloromethyl-4-nitrophenol to obtain corresponding salts of 3*H-*indolium which further are treated under basic conditions
**characterised i n** that said method comprises the following steps:
i) alkalizing a solution of corresponding 3H-indolium salt to obtain enamine of general formula (II) 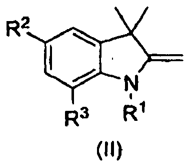
ii) treating enamine obtained of the formula (II) with 2-chloromethyl-4-nitrophenol to obtain a chloride of general formula (III) 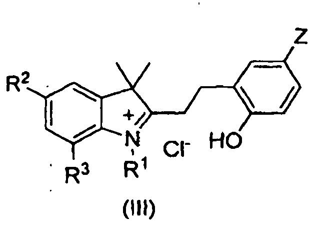
iii) purifying the chloride of formula (III) obtained in the step ii) by recrystallization or chromatographically, following by optional dissoluting and precipitating under neutral or weakly basic conditions to obtain the target compounds of formula (I);
iv) if desired, recrystallizing from acetonitrile the target compounds of formula (I).

14. The method according to claim 13, **characterised in that**
- to obtain the compounds of formula (I), wherein R¹ is methyl, ethyl or benzyl; R² is hydrogen, methyl or Br; R³ is hydrogen or methyl, and Z is nitro group, precipitation of step iii) is carried out by adding dropwise an aqueous solution of a base, such as ammonia; or
- to obtain the compounds of formula (I), wherein R¹ is allyl; R²-R⁴ are hydrogen and Z is a nitro group, no base is added in step iii).

15. The method according to claim 13 or 14, **characterised in that** as the suitable 3*H*-indolium salt in step i) is used, for example, iodide or perchlorate; sodium carbonate is used for alkalization; and reaction mixture of step ii) is stirred for up to 3 hours at temperature of 20°C-80°C and then optionally held for at least 12 hours at 5°C ± 1°C temperature.

## Patentansprüche

1. Die Verbindungen der allgemeinen Formel (I) 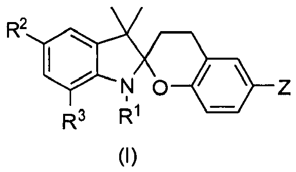 wobei
R¹ C₁-C₃-Alkyl, ω-funkzionalisiertes Alkyl, Alyl oder Benzyl bezeichnet;
R² Wasserstoff, Methyl, F, Cl, Br, Phenyl, 2-Phenylethen-1-yl, Phenylethinyl bezeichnet;
R³ Wasserstoff oder Methyl bezeichnet;
Z eine Nitrogruppe bezeichnet.

2. Die Verbindungen der allgemeinen Formel(I) nach Anspruch 1, wobei R¹ Methyl, Ethyl, Alyl oder Benzyl bezeichnet; R² Wasserstoff, Methyl, Brom bezeichnet; R³ Wasserstoff oder Methyl darstellt , Z eine Nitrogruppe bezeichnet.

3. Die Verbindung nach Ansprucht 1 oder 2, die 1',3',3'-trimethyl -6-nitro-1',3,3',4-tetrahydrospiro[chromen-2,2'-indol] ist **(4a).**

4. Die Verbindung nach Ansprucht 1 oder 2, die 1',3',3',5'-tetramethyl-6-nitro-1',3,3',4-tetrahydrospiro[chromen-2,2'-indol] ist **(4b).**

5. Die Verbindung nach Ansprucht 1 oder 2, die 5'-brom-1',3',3'-trimethyl-6-nitro-1',3,3',4-tetrahydrospiro[chromen-2,2'-indol] ist **4c).**

6. Die Verbindung nach Ansprucht 1 oder 2, die 1',3',3',7'-tetramethyl-6-nitro-1',3,3',4-tetrahydrospiro[chromen-2,2'-indol] ist **(4d).**

7. Die Verbindung nach Ansprucht 1 oder 2, die 1'-ethyl-3',3'-dimethyl-6-nitro-1',3,3',4-tetrahydrospiro[chromen-2,2'-indol] ist **(4e).**

8. Die Verbindung nach dem Punkt 1 oder 2, die 1'-alyl)-3',3'-dimethyl-6-nitro-1',3,3',4-tetrahydrospiro[chromen-2,2'-indol] ist **(4f).**

9. Die Verbindung nach Ansprucht 1 oder 2, die 1'-benzyl-3',3'-dimethyl-6-nitro-1',3,3',4-tetrahydrospiro[chromen-2,2'-indol] ist **(4g).**

10. Die Verbindung nach irgendwelchem früheren Ansprucht, die sich mit photochromischen Eigenschaften ausprägt.

11. Die Verwendung der Verbindungen nach irgendwelchem Ansprucht 1-9 ist als moleküle Photoverbinder.

12. Die Verbindungen, deren allgemeine Formel (III), 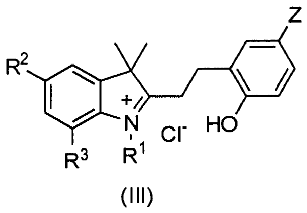 wobei R¹ Methyl oder Ethyl bezeichnet; R² Wasserstoff, Methyl oder Brom bezeichnet; R³ Wasserstoff oder Methyl bezeichnet und Z eine Nitrogruppe bezeichnet, sind als Zwischenverbindungen, um die Verbindungen nach dem Punkt 3-7 zu erhalten.

13. Die Verbindungen (I), deren Formel (I) 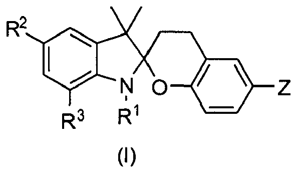 nach irgendwelchem Ansprucht 1-9, in denen R¹ Methyl, Ethyl, Alyl oder Benzyl bezeichnet; R² Wasserstoff, Methyl, Brom bezeichnet; R³ Wasserstoff oder Methyl bezeichnet, und Z eine Nitrogruppe ist, die Weise der Herstellung, wo man Derivate der 1-Substitutierten-3,3-dimethyl-2-methylenindol mit 2-Chlormethyl-4-nitrophenol alkiliert und erhaltene endsprechende 3*H*-indoliumssalze, die man mit Lauge bearbeitet;
**unterscheidet sich dadurch, dass**
i) entsprechende Lösung des 3H-indoliumssalzes alkalisiert, um Enamin der Formel (II) zu erhalten 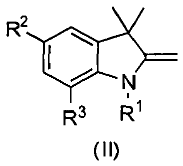
ii) das erhaltene Enamin der Formel (II) wirkt man mit 2-chlormethyl-4-nitrofenol, um Chlorid der allgemeinen Formel (III) zu erhalten 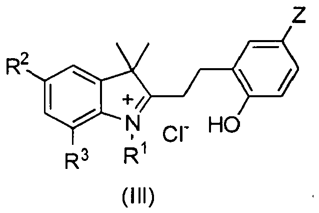
iii) im Stadium ii) das erhaltene Chlorid der Formel (III) kristalisiert man um oder reinigt man chromatografisch, danach nicht unbedingt löst man und unterschlägt im neutralen oder schwach alkalischen Medium, um gezielte Verbindungen der Formel (I) zu erhalten;
iv)wenn es nötig ist, erhaltene gezielte Verbindungen der Formel (I) kristalisiert man aus Acetonitril um .

14. Die Weise nach Ansprucht 13 **unterscheidet sich dadurch,** dass um die Verbindungen nach der Formel (I) zu erhalten, wobei R¹ Methyl, Ethyl oder Benzil bezeichnet; R² Wasserstoff, Methyl, Brom bezeichnet; R³ Wasserstoff oder Methyl bezeichnet, ir Z eine Nitrogruppe bezeichnet, den Niederschlag des Stadiums iii) vollzieht man die Lösung der Lauge z.B.Ammoniak zu träufeln ; oder - um Verbindung der Formel (I) zu erhalten, wobei R¹ Alyl bezeichnet; R²-R⁴ Wasserstoff bezeichnet und Z eine Nitrogruppe bezeichnet, im Stadium iii) gebraucht man keine Lauge.

15. Die Weise nach Ansprucht 13 oder 14 **unterscheidet sich dadurch,** dass im Stadium i) passendes 3H-indolsalz z.B. Jodid oder Perchlorat ist. Um zu alkalisieren, gebraucht man Karbonat Natriums; und die Mischung der Reaktion des Stadiums ii) mischt man bis 3 Stunden lang unter der Temperatur 20°C-80°C und dann hält man mindestens 12 Stunden unter der Temperatur 5°C ± 1°C.

## Revendications

1. Les composés de formule générale (I) 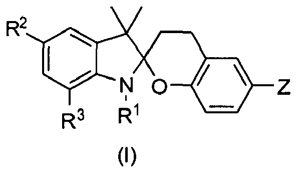 dans laquelle:
R¹ représente un alkyle C₁-C₃, un groupe alkyle ω-fonctionnalisé, un groupe allyle ou un groupe benzyle;
R² représente un atome d'hydrogène, un groupe méthyle, F, CI, Br, un groupe phényle, 2-fenileten-1-yle, phényléthynyle;
R³ un atome d'hydrogène ou un groupe méthyle;
Z représente le groupe nitro.

2. Les composés de formule générale (I) selon la revendication 1, dans lequel:
R¹ représente un groupe méthyle, éthyle, allyle ou benzyle; R² représente un atome d'hydrogène, un groupe méthyle, le brome; R³ un atome d'hydrogène ou un groupe méthyle, Z représente le groupe nitro.

3. Un composé selon la revendication 1 ou la revendication 2, lequel est 1',3',3'-triméthyl-6-nitro-1',3,3',4-tetrahydrospiro[chromène-2,2'-indole] **(4a).**

4. Un composé selon la revendication 1 ou la revendication 2, lequel est 1',3',3',5'-tetraméthyl-6-nitro-1',3,3',4-tetrahydrospiro[chromène-2,2'-indole] **(4b).**

5. Un composé selon la revendication 1 ou la revendication 2, lequel est 5'-bromo-1',3',3'-triméthyl-6-nitro-1',3,3',4-tetrahydrospiro[chromène-2,2'-indole] **(4c).**

6. Un composé selon la revendication 1 ou la revendication 2, lequel est 1',3',3',7'-tetraméthyl-6-nitro-1',3,3',4-tetrahydrospiro[chromène-2,2'-indole] **(4d).**

7. Un composé selon la revendication 1 ou la revendication 2, lequel est 1'-éthyl-3',3'-diméthyl-6-nitro-1',3,3',4-tetrahydrospiro[chromène-2,2'-indole] **(4e).**

8. Un composé selon la revendication 1 ou la revendication 2, lequel est 1'-allyl-3',3'-diméthyl-6-nitro-1',3,3',4-tetrahydrospiro[chromène-2,2'-indole] **(4f).**

9. Un composé selon la revendication 1 ou la revendication 2, lequel est 1'-benzyl-3',3'-diméthyl-6-nitro-1',3,3',4-tetrahydrospiro[chromène-2,2'-indole] **(4g).**

10. Un composé photochromique selon l'une quelconque des revendications précédentes.

11. L'application des composés selon l'une quelconque des revendications 1 à 9 en tant que photo-interrupteurs moléculaire.

12. Les composés de formule générale (III), 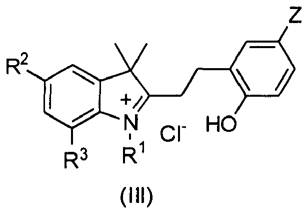 dans laquelle R¹ représente un groupe méthyle ou éthyle; R² représente un atome d'hydrogène, un groupe méthyle ou bromo; R³ représente un atome d'hydrogène ou un groupe méthyle et Z représente le groupe nitro, en tant qu'intermédiaires de composés obtenus selon les revendications 3-7.

13. Les composés (I) de formule (I) 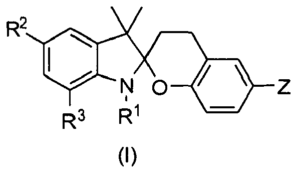 selon l'une quelconque des revendications 1-9, dans lequel R¹ représente un groupe méthyle, éthyle, allyle ou benzyle; R² représente un atome d'hydrogène, un groupe méthyle ou bromo; R³ représente un atome d'hydrogène ou un groupe méthyle, et Z est un groupe nitro, le processus dans lequel l'alkylation de dérivés 3,3-diméthyl-2-mehylènindole, substitués en position 1, par 2-chlorométhyl-4-nitrophénol conduit aux sels de 3H-indolium correspondants, lesquels sont traités par la base
**qui diffère par ce,** que
i) pour former l'énamine de formule (II), la solution du sel correspondant de 3H-indolium est rendue alcaline 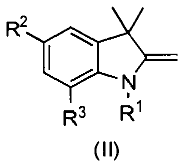
ii) l'énamine de formule (II) obtenu, est mis en réaction avec 2-chlorométhyl-4-nitrophénol, pour conduire au chlorure de formule générale (III) 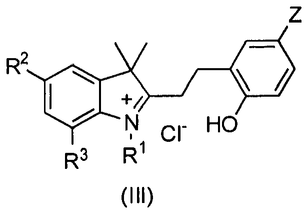
iii) le chlorure de formule (III) obtenu à l'étape ii) est cristallisé ou purifié par chromatographie, puis éventuellement dissout et précipité dans les conditions soit neutres, soit légèrement alcalines pour former des composés-cibles de formule (I);
iv) s'il est nécessaire, les composés-cibles de formule (I) sont recristallisés dans l'acétonitrile.

14. Le procédé selon la revendication 13, **diffère par ce**, que
- pour former les composés de formule (I), dans lesquels R¹ représente un groupe méthyle, éthyle ou benzyle; R² représente un atome d'hydrogène, un groupe méthyle, le brome; R³ représente un atome d'hydrogène ou un groupe méthyle, et Z représente le groupe nitro, la précipitation de l'étape iii) est effectué par l'ajout d'une solution aqueuse d'alcali tel que l'ammoniaque; ou
- pour former le composé de formule (I), dans lequel R¹ représente un groupe allyle; R²-R⁴ représente un atome d'hydrogène et Z représente le groupe nitro, à l'étape iii) l'alcali n'est pas utilisé.

15. Le procédé selon la revendication 13 ou la revendication 14, **diffère par c e ,** que à l'étape i) le sel de 3H-indolium approprié est, par exemple, l'iodure ou le perchlorate, et pour le traitement alcalin le carbonate de sodium est utilisé; et le mélange réactionnel de l'étape ii) est agité jusqu'à 3 heures à température 20°C-80°C puis éventuellement conserve au moins 12 heures à température 5°C ± 1°C.
